# EUROPEAN PATENT SPECIFICATION

(11) **EP 3 302 103 B1**
(45) Date of publication and mention of the grant of the patent: **12.12.2018**
(21) Application number: 16726382.1
(22) Date of filing: 25.05.2016
(51) Int. Cl.: A23L 33/135, A23L 33/21

(54) **COMPOSITIONS AND METHODS OF PRODUCTION THEREOF**
ZUSAMMENSETZUNGEN UND VERFAHREN ZUR HERSTELLUNG DAVON
COMPOSITIONS ET LEURS PROCÉDÉS DE PRODUCTION

(30) Priority: 27.05.2015 GB 201509023
(43) Date of publication of application: 11.04.2018
(73) Proprietor: Optibiotix Limited, Heslington, York YO10 5DG (GB)
(72) Inventor: O'HARA, Stephen Patrick, York Yorkshire YO10 5DG (GB); KOLIDA, Sofia, York Yorkshire YO10 5DG (GB)
(74) Representative: Appleyard Lees IP LLP
(86) International application number: PCT/GB2016/051516
(87) International publication number: WO 2016/189306

(56) References cited:
- EP-A1- 1 374 878
- WO-A1-2004/052121
- WO-A1-2014/110685
- WO-A1-2015/067938
- WO-A1-2015/067949

## Description

### Technical Field of the Invention

The invention relates to a prebiotic composition which is selective for the growth of *Propionibacterium* bacterial strain(s), for use in, but not limited to, promoting propionate production in the gut so as to regulate appetite in an individual.

### Background to the Invention

Prebiotics are dietary ingredients which can selectively enhance the levels and/or activity of beneficial indigenous gut microbiota, such as lactobacilli or bifidobacteria, and they are finding much increased application in the food sector. Prebiotics are non-digestible food ingredients that are selectively metabolised by colonic bacteria which contribute to improved health. As such, their use can promote beneficial changes within the indigenous gut microbial milieu and they can therefore help survivability of probiotics. They are distinct from most dietary fibres like pectin, celluloses, xylan, which are not selectively metabolised in the gut. Criteria for classification as a prebiotic is that it must resist gastric acidity, hydrolysis by mammalian enzymes and absorption in the upper gastrointestinal tract, it is fermented by intestinal microflora and selectively stimulates the growth and/or activity of intestinal bacteria associated with health and well-being.

Increasing colonic levels of propionate is believed to help regulate appetite in an individual. However it is difficult to administer propionate directly to the large intestine due to the destructive digestive environment and the absorptive capacity of the upper gastrointestinal tract.

Fructo-oligosaccharides (FOS, inulin and oligofructose) and galactooligosaccharides (GOS) have been demonstrated to fulfil the criteria for prebiotic classification repeatedly in human intervention studies. Currently available fructooligosaccharides and galactooligosaccharides target the growth and/or activity of bifidobacteria and lactobacilli, neither of which can produce propionate. Currently, there is no known selective prebiotic for *Propionibacterium* WO 2015/067949 A1 relates to a prebiotic composition which is selective for the growth of Propionibacterium bacterial strain(s), for use in, but not limited to, promoting propionate production in the gut so as to regulate appetite in an individual.

It is an object of the present invention to provide a prebiotic composition which allows for the specific growth of a propionate producing bacteria. It would also be desirable if the prebiotic targeted a beneficial species or strain of *Propionibacterium.*

### Summary of the Invention

In accordance with a first aspect of the present invention, there is provided a prebiotic composition comprising a galacto oligosaccharide (GOS) produced from one or more *Propionibacterium* bacterial strains, wherein the GOS acts as a selective growth medium for *Propionibacterium* bacterial strains, and wherein the GOS is substantially the same as the form produced by reverse β-galactosidase reaction from *Propionibacterium* bacterial strains.

Preferably, the GOS is produced and/or is selective for one of more of the following bacterial strains: *Propionibacterium jensenii; Propionibacterium freudenreichii; Propionibacterium acidipropionici,* or sub-species or mutant strains thereof.

The GOS may be produced from the selected *Propionibacterium* bacterial genera or strains and the GOS may act as a selective growth medium for said selected *Propionibacterium* bacterial genera or strain.

The prebiotic composition will preferably be present in the composition in an effective amount so as to elicit a positive and gradual change in the proportions and activity of *Propionibacterium* in the gut. Higher amounts may be utilised if change in the microbiota is required quickly or if the composition is being used to help seed the gut with a new bacterial strain not currently present.

The prebiotic composition may be encapsulated. Many encapsulation techniques will be apparent to the skilled addressee and the one employed will be tailored to the required stability of the prebiotic growth medium during digestive transit.

The prebiotic composition may further comprise an excipient or carrier compound to enable it to pass through at least part of the gastrointestinal environment of the body and be efficiently delivered to, and released in the lower gut. The prebiotic may be concentrated and/or freeze dried. The composition may be in a number of formats, such as in the form of a liquid (which may be drinkable) and/or powder which can be mixed with a solid or liquid food stuff.

The prebiotic composition may be combined with one or more active ingredients, such as vitamins, minerals, phytochemicals, antioxidants, probiotic bacterial strains and combinations thereof.

Vitamins may include fat soluble vitamins such as vitamin A, vitamin D, vitamin E, and vitamin and combinations thereof. In some embodiments, vitamins can include water soluble vitamins such as vitamin C (ascorbic acid), the B vitamins (thiamine or B 1, riboflavoin or B25 niacin or B3, pyridoxine or B6, folic acid or B9, cyanocobalamin or B12, pantothenic acid, biotin), and combinations thereof.

Minerals may include but are not limited to sodium, magnesium, chromium, iodine, iron, manganese, calcium, copper, fluoride, potassium, phosphorous, molybdenum, selenium, zinc, and combinations thereof.

Antioxidants may include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

Phytochemicals may include but are not limited to cartotenoids, chlorophyll, chlorophyllin, fiber, flavanoids, anthocyanins, cyanidin, delphinidin, malvidin, pelargonidin, peonidin, petunidin, flavanols, catechin, epicatechin, epigallocatechin, epigallocatechin gallate, theaflavins, thearubigins, proanthocyanins, flavonols, quercetin, kaempferol, myricetin, isorhamnetin, flavonones hesperidin, naringenin, eriodictyol, tangeretin, flavones, apigenin, luteolin, lignans, phytoestrogens, resveratrol, isoflavones, daidzein, genistein, glycitein, soy isoflavones, and combinations thereof.

Probiotic strains may also be incorporated into the composition. It is preferred that the probiotic strains comprise *Propionibacterium* bacterial strains. It is most preferred that probiotic strain or strains comprise the *Propionibacterium* bacterial strain or strains used to initially produce the GOS.

In accordance with a further aspect of the present invention, there is provided a prebiotic composition for use in the regulation and/or modulation of appetite. Alternatively or additionally, the composition may be for use in the management or treatment of obesity and/or weight management. The composition comprising a galactooligosaccharide (GOS) produced from one or more *Propionibacterium* bacterial strains, wherein the GOS acts as a selective growth medium for *Propionibacterium* bacterial strains, and wherein the GOS is substantially the same as the form produced by reverse β-galactosidase reaction from *Propionibacterium* bacterial strains may be for use as a medicament or pharmaceutical and/or a dietary supplement.

In accordance with a further aspect of the present invention, there is provided a prebiotic composition for use in the treatment of obesity, the composition comprising a galactooligosaccharide (GOS) produced from one or more *Propionibacterium* bacterial strains, wherein the GOS acts as a selective growth medium for *Propionibacterium* bacterial strains, and wherein the GOS is substantially the same as the form produced by reverse β-galactosidase reaction for *Propionibacterium* bacterial strains.

In a yet further aspect of the present invention, there is provided a use of a prebiotic composition, in the manufacture of a medicament for use in the treatment or management of obesity, the composition comprising a microbially produced oligosaccharide, wherein the composition comprises a galactooligosaccharide (GOS) produced from one or more *Propionibacterium* bacterial strains, wherein the GOS acts as a selective growth medium for *Propionibacterium* bacterial strains, and wherein the GOS is substantially the same as the form produced by reverse β-galactosidase reaction for *Propionibacterium* bacterial strains.

Alternative (or additionally) to a pharmaceutical or medicament, the composition may be used as a dietary supplement, a nutraceutical or a functional food. A yet further aspect of the present invention may be a prebiotic composition for use as a dietary supplement, a nutraceutical or a functional food, the composition comprising a galacto oligosaccharide (GOS) produced from one or more *Propionibacterium* bacterial strains, wherein the GOS acts as a selective growth medium for *Propionibacterium* bacterial strains, and wherein the GOS is substantially the same as the form produced by reverse β-galactosidase reaction for *Propionibacterium* bacterial strains.

It will be apparent to the skilled addressee that the features of the prebiotic composition in connection with the first aspect of the invention will also be applicable and interchangeable for the composition for use as a pharmaceutical, medicament, dietary supplement, nutraceutical or functional food.

Furthermore, the composition could be incorporated into an existing food, such as yoghurt or as a powder which can be easily blended with foodstuffs or made into a liquid drink.

In a further aspect of the present invention, there is provided a composition for use in increasing propionate levels in the lower gut of an individual wherein the composition promotes the growth of propionate secreting bacteria.

In another aspect of the present invention, there is provided a method of producing galactooligosaccharide (GOS) comprising the steps of growing one or more *Propionibacterium* strains in a growth medium comprising up to 50 % lactose at a temperature of up to 55 °C for up to 24 hours under anaerobic conditions and harvesting GOS from the *Propionibacterium* cells.

Preferably, the one or more the one or more *Propionibacterium* strains are grown in a growth medium comprising up to 40 % lactose at a temperature of up to 50 °C for up to 24 hours. The one or more *Propionibacterium* strains may be grown in a growth medium comprising in the range of about 20 to about 40 % lactose at a temperature in the range of about 35 to about 50°C for about 10 to about 14 hours.

The GOS may be harvested by a number of methods, but it is preferred that is harvested from the cells by lysis. Such a lysis may involve one or more freeze-thawing steps.

The *Propionibacterium* strains may be selected from one or more of the following: *Propionibacterium jensenii; Propionibacterium freudenreichii; Propionibacterium acidipropionici,* or sub-species or mutant strains thereof.

Preferably, the method of producing GOS in a selected *Propionibacterium* strain(s) is optimised.

The method as hereinabove described, may be used to produce GOS for use in a compositional aspects of the present invention.

### Detailed Description of the Invention

Embodiments of the present invention will now be described, by way of example only, in which:
Figure 1 is a graph showing the ratio between the most prevalent GOS species for *P. jensenii* calculated at different temperatures and timepoints;
Figure 2 is a graph showing the two GOS formation rates at 30°C and 50°C of the selected two *Propionibacterium* strains;
Figure 3 is a graph showing the ratio of the actual GOS formation rate over the theoretical GOS formation rate at 30°C and 50°C for the selected strains;
Figure 4 is a graph showing the analysis of the difference in β-galactosidase activity from the initial screening experiments and the later GOS synthesis experiments in the selected strains; and
Figure 5 shows the analysis (by means of the Log/Stat ratio) of the dependency of expression of β-galactosidase of the selected strains.

Mechanistically glycosidases are all transferases that use water as their preferred acceptor molecule. Under appropriate circumstance, however, such as high concentrations of substrate carbohydrate, these enzymes will transfer monosaccharide moieties from the substrate (acting as glycosyl donor) to other substrate or non-substrate carbohydrates (acting as glycosyl acceptor). Typically, the products of these reactions are complex mixtures containing all possible glycosidic linkages but in differing amounts. As the reactions are kinetically controlled, the linkage profile synthesised should map onto the rate constants for hydrolysis of those linkages by the producing enzyme. Consequently the oligosaccharides may be more readily metabolised by the producing organisms than by others in the gastrointestinal ecosystem. This approach has shown promise in laboratory testing.

It is possible, however in many enzyme synthesis reactions to include other carbohydrates which will act as acceptors in addition to the lactose. In this way, novel mixtures containing novel structures could be built up.

The basis of the present experiments was to reversibly use β-galactosidases in *Propionibacterium* strains so as to produce a novel GOS. Ordinarily, β-galactosidases would hydrolyse lactose. However, by changing the reaction conditions, in terms of substrate concentration and temperature, the enzyme acts reversibly and generates an oligosaccharide version of the lactose (GOS).

### EXPERIMENTS

Experiments were conducted in two phases. The first phase screened 77 strains for the detection of β-galactosidase hydrolytic activity based on the breakdown of *ortho-*Nitrophenyl-β-galactoside (ONPG). Growth conditions were adjusted to attempt to improve the overall growth characteristics. Total β-galactosidase activity was assessed and strains exhibiting the highest activity were then put forward to the second phase. During the second phase, a feasibility study was conducted to screen the selected strains for their actual ability to synthesise GOS.

Screening of 77 *Propionibacterium* strains was conducted for the detection of β-galactosidase hydrolytic activity based on the breakdown of ONPG. The total β-galactosidase activity was assessed in miller units.

### β-galactosidase activity in Propionibacterium

A range of *Propionibacterium* strains (including different species and sub-species) were pre-grown from a -80°C stock for 72 hours at 30°C in 200 µl LB medium supplemented with 1% glucose in a standard 96 wells-plate. Cultures were re-diluted 100 fold to 1600 µl LB supplied with 1% glucose deep-well plates. Growth was performed in anaerobic conditions at 37°C for 96 hours OD₆₀₀ was determined after a 10-fold dilution of the cultures. To assess β-galactosidase activity, cells were first centrifuged at 5000 x g at 4°C. Then the pellets were lysed using 0.5 gram silicabeads (0.1mm) in 800 µl 0.05M NaPi buffer pH=7.0. The supernatant was used for determining the β-galactosidase activity at 30°C using a standard protocol.

Table 1 below illustrates the results of those *Propionibacterium* strains which were screened using the above protocol.

**Table 1**

| **Strain no.** | **Total Bgal activity** | | **Average OD600** | | **Bgal activity (Miller Units)** | | | |
|---|---|---|---|---|---|---|---|---|
| | **Average** | **Stdev** | **Average** | **Stdev** | **Average** | **Stdev** | **Species** | **SubSpecies** |
| **no.** | **µmol/min/l** | | **AU** | | **µmol/min/OD-Unit** | | | |
| 4204 | 17.6 | 3.9 | 2.94 | 0.10 | 6.0 | 1.5 | *Propionibacterium acidipropionici* | |
| 380 | 15.8 | 0.7 | 2.86 | 0.34 | 5.6 | 0.4 | *Propionibacterium sp.* | |
| 2166 | 0.9 | 0.3 | 0.18 | 0.03 | 4.8 | 0.7 | *Propionibacterium freudenreichii* | *freudenreichii* |
| 359 | 7.9 | 4.0 | 1.77 | 0.90 | 4.5 | 0.1 | *Propionibacterium sp.* | |
| 1134 | 10.6 | 1.0 | 2.49 | 0.07 | 4.2 | 0.3 | *Propionibacterium freudenreichii* | *shermanii* |
| 364 | 10.8 | 1.3 | 2.91 | 0.03 | 3.7 | 0.4 | *Propionibacterium jensenii* | |
| 2060 | 8.7 | 1.1 | 2.45 | 0.22 | 3.5 | 0.1 | *Propionibacterium freudenreichii* | *shermanii* |
| 4199 | 5.9 | 1.4 | 1.69 | 0.44 | 3.5 | 0.1 | *Propionibacterium acidipropionici* | |
| 4201 | 6.4 | 0.3 | 1.92 | 0.02 | 3.3 | 0.1 | *Propionibacterium acidipropionici* | |
| 2175 | 6.8 | 0.2 | 2.04 | 0.06 | 3.3 | 0.2 | *Propionibacterium freudenreichii* | *freudenreichii* |
| 2145 | 8.0 | 0.6 | 2.39 | 0.01 | 3.3 | 0.2 | *Propionibacterium freudenreichii* | *freudenreichii* |
| 2168 | 7.3 | 1.2 | 2.36 | 0.07 | 3.1 | 0.6 | *Propionibacterium freudenreichii* | *freucfenreichii* |
| 2174 | 4.5 | 2.9 | 1.33 | 0.68 | 3.1 | 0.6 | *Propionibacterium freudenreichii* | *freudenreichii* |
| 2173 | 6.4 | 0.4 | 2.06 | 0.08 | 3.1 | 0.3 | *Propionibacterium freudenreichii* | *freudenreichii* |
| 384 | 1.5 | 0.3 | 0.53 | 0.11 | 2.9 | 1.1 | *Propionibacterium freudenreichii* | *freudenreichii* |
| 2171 | 7.7 | 3.5 | 3.00 | 0.00 | 2.6 | 1.2 | *Propionibacterium freudenreichii* | *freudenreichii* |
| 362 | 5.7 | 0.6 | 2.38 | 0.08 | 2.4 | 0.3 | *Propionibacterium acidipropionici* | |
| 2172 | 4.4 | 2.2 | 1.83 | 0.34 | 2.3 | 0.8 | *Propionibacterium freudenreichii* | *freudenreichii* |
| 360 | 0.6 | 0.2 | 0.14 | 0.22 | 2.2 | 0.3 | *Propionibacterium freudenreichii* | *shermanii* |
| 2156 | 4.4 | 1.8 | 2.01 | 0.46 | 2.1 | 0.4 | *Propionibacterium freudenreichii* | *freudenreichii* |
| 2541 | 3.2 | 2.7 | 1.65 | 0.18 | 2.1 | 1.9 | *Propionibacterium sp.* | |
| 2149 | 5.4 | 3.0 | 2.70 | 0.04 | 2.0 | 1.1 | *Propionibacterium freudenreichii* | *freudenreichii* |
| 375 | 5.4 | 0.5 | 3.00 | 0.00 | 1.8 | 0.2 | *Propionibacterium acidipropionici* | |
| 2169 | 4.7 | 0.6 | 2.68 | 0.05 | 1.8 | 0.2 | *Propionibacterium freudenreichii* | *freudenreichii* |
| 2146 | 3.4 | 0.2 | 2.37 | 0.33 | 1.4 | 0.1 | *Propionibacterium freudenreichii* | *freudenreichii* |
| 374 | 3.2 | 5.4 | 2.42 | 0.01 | 1.3 | 2.2 | *Propionibacterium freudenreichii* | *shermanii* |
| 2167 | 2.9 | 0.6 | 2.23 | 0.06 | 1.3 | 0.2 | *Propionibacterium freudenreichii* | *freudenreichii* |
| 2160 | 3.2 | 1.1 | 2.63 | 0.29 | 1.2 | 0.3 | *Propionibacterium freudenreichii* | *freudenreichii* |
| 2150 | 0.9 | 0.5 | 1.24 | 1.04 | 1.1 | 0.7 | *Propionibacterium freudenreichii* | *freudenreichii* |
| 2159 | 2.7 | 1.1 | 2.33 | 0.30 | 1.1 | 0.3 | *Propionibacterium freudenreichii* | *freudenreichii* |
| 2543 | 2.1 | 0.3 | 1.88 | 0.01 | 1.1 | 0.2 | *Propionibacterium sp.* | |
| 371 | 1.2 | 0.0 | 1.10 | 0.13 | 1.1 | 0.1 | *Propionibacterium freudenreichii* | *shermanii* |
| 4200 | 2.6 | 0.4 | 2.37 | 0.11 | 1.1 | 0.1 | *Propionibacterium acidipropionici* | |
| 2178 | 1.2 | 0.2 | 1.33 | 0.54 | 1.1 | 0.6 | *Propionibacterium freudenreichii* | *freudenreichii* |
| 2164 | 2.4 | 0.5 | 2.28 | 0.04 | 1.1 | 0.3 | *Propionibacterium freudenreichii* | *freudenreichii* |
| 2162 | 2.4 | 1.9 | 2.04 | 1.23 | 1.0 | 0.3 | *Propionibacterium freudenreichii* | *freudenreichii* |
| 367 | 2.0 | 0.5 | 1.93 | 0.08 | 1.0 | 0.2 | *Propionibacterium freudenreichii* | *shermanii* |
| 2068 | 2.2 | 0.3 | 2.13 | 0.12 | 1.0 | 0.1 | *Propionibacterium freudenreichii* | *shermanii* |
| 2177 | 1.9 | 0.3 | 1.86 | 0.12 | 1.0 | 0.1 | *Propionibacterium freudenreichii* | *freudenreichii* |
| 2165 | 2.4 | 1.0 | 2.36 | 0.02 | 1.0 | 0.4 | *Propionibacterium freudenreichii* | *freudenreichii* |
| 2155 | 2.7 | 0.1 | 2.65 | 0.11 | 1.0 | 0.1 | *Propionibacterium freudenreichii* | *freudenreichii* |
| 2069 | 1.9 | 0.7 | 1.86 | 0.69 | 1.0 | 0.1 | *Propionibacterium freudenreichii* | *shermanii* |
| 2161 | 2.2 | 1.3 | 2.17 | 0.31 | 1.0 | 0.5 | *Propionibacterium freudenreichii* | *freudenreichii* |
| 2066 | 2.4 | 0.2 | 2.47 | 0.15 | 1.0 | 0.0 | *Propionibacterium freudenreichii* | *freudenreichii* |
| 365 | 2.4 | 0.2 | 2.50 | 0.03 | 0.9 | 0.1 | *Propionibacterium freudenreichii* | *shermanii* |
| 2544 | 2.2 | 0.4 | 2.32 | 0.02 | 0.9 | 0.2 | *Propionibacterium sp.* | |
| 2158 | 2.1 | 1.2 | 2.24 | 0.06 | 0.9 | 0.5 | *Propionibacterium freudenreichii* | *freudenreichii* |
| 361 | 2.7 | 2.1 | 2.96 | 0.09 | 0.9 | 0.7 | *Propionibacterium thoenni* | *shermanii* |
| 2163 | 1.9 | 0.9 | 2.11 | 0.20 | 0.9 | 0.3 | *Propionibacterium freudenreichii* | *freudenreichii* |
| 2154 | 2.2 | 0.2 | 2.56 | 0.16 | 0.9 | 0.0 | *Propionibacterium freudenreichii* | *freudenreichii* |
| 382 | 2.1 | 0.9 | 2.41 | 0.83 | 0.8 | 0.1 | *Propionibacterium sp.* | |
| 379 | 0.9 | 0.4 | 1.09 | 0.07 | 0.8 | 0.4 | *Propionibacterium freudenreichii* | *freudenreichii* |
| 2542 | 1.9 | 1.2 | 2.32 | 0.05 | 0.8 | 0.5 | *Propionibacterium sp.* | |
| 372 | 1.6 | 0.4 | 2.25 | 0.04 | 0.7 | 0.2 | *Propionibacterium freudenreichii* | *shermanii* |
| 2157 | 2.0 | 1.3 | 2.61 | 0.38 | 0.7 | 0.4 | *Propionibacterium freudenreichii* | *freudenreichii* |
| 369 | 1.4 | 0.1 | 2.04 | 0.04 | 0.7 | 0.1 | *Propionibacterium freudenreichii* | *shermanii* |
| 1256 | 1.7 | 0.9 | 2.40 | 0.29 | 0.7 | 0.3 | *Propionibacterium sp.* | |
| 2144 | 1.0 | 0.2 | 0.93 | 1.11 | 0.6 | 0.0 | *Propionibacterium freudenreichii* | *freudenreichii* |
| 2179 | 1.3 | 0.3 | 2.12 | 0.03 | 0.6 | 0.1 | *Propionibacterium freudenreichii* | *freudenreichii* |
| 2170 | 1.4 | 0.2 | 2.33 | 0.04 | 0.6 | 0.1 | *Propionibacterium freudenreichii* | *freudenreichii* |
| 2336 | 1.4 | 0.8 | 2.48 | 0.05 | 0.6 | 0.3 | *Propionibacterium freudenreichii* | *shermanii* |
| 2181 | 1.0 | 0.2 | 1.82 | 0.36 | 0.5 | 0.0 | *Propionibacterium freudenreichii* | *freudenreichii* |
| 2176 | 1.6 | 0.1 | 3.13 | 0.15 | 0.5 | 0.0 | *Propionibacterium freudenreichii* | *freudenreichii* |
| 2147 | 1.1 | 0.5 | 2.10 | 0.67 | 0.5 | 0.1 | *Propionibacterium freudenreichii* | *freudenreichii* |
| 370 | 1.0 | 0.2 | 2.05 | 0.06 | 0.5 | 0.1 | *Propionibacterium freudenreichii* | *shermanii* |
| 383 | 0.8 | 0.3 | 1.87 | 0.80 | 0.5 | 0.3 | *Propionibacterium freudenreichii* | *freudenreichii* |
| 2663 | 1.2 | 0.7 | 2.60 | 0.04 | 0.5 | 0.3 | *Propionibacterium freudenreichii* | *shermanii* |
| 2182 | 1.2 | 0.8 | 2.62 | 0.09 | 0.5 | 0.3 | *Propionibacterium freudenreichii* | *freudenreichii* |
| 2151 | 1.0 | 0.1 | 2.28 | 0.26 | 0.4 | 0.1 | *Propionibacterium freudenreichii* | *freudenreichii* |
| 2143 | 0.5 | 0.0 | 1.14 | 0.06 | 0.4 | 0.0 | *Propionibacterium freudenreichii* | *freudenreichii* |
| 2152 | 0.9 | 0.1 | 2.45 | 0.19 | 0.4 | 0.1 | *Propionibacterium freudenreichii* | *freudenreichii* |
| 2007 | 0.9 | 0.2 | 2.40 | 0.04 | 0.4 | 0.1 | *Propionibacterium freudenreichii* | *shermanii* |
| 2065 | 0.9 | 0.1 | 2.39 | 0.11 | 0.4 | 0.0 | *Propionibacterium freudenreichii* | *shermanii* |
| 363 | 0.6 | 0.1 | 1.66 | 0.07 | 0.3 | 0.1 | *Propionibacterium freudenreichii* | *freudenreichii* |
| 2148 | 0.7 | 0.2 | 1.97 | 0.07 | 0.3 | 0.1 | *Propionibacterium freucfenreichii* | *freucfenreichii* |
| 2180 | 1.0 | 0.1 | 3.00 | 0.00 | 0.3 | 0.0 | *Propionibacterium freudenreichii* | *freudenreichii* |
| 2067 | 0.6 | 0.1 | 1.92 | 0.01 | 0.3 | 0.0 | *Propionibacterium freudenreichii* | *shermanii* |
| 1219 | 0.4 | 0.2 | 2.27 | 0.06 | 0.2 | 0.1 | *Propionibacterium freudenreichii* | *freudenreichii* |

The highest β-galactosidase expressing strains were then included in the next phase of the study.

### Analysis of GOS Production in the Chosen Strains

The following growth protocols were used:
*Propionibacterium* strains were pre-grown from the -80°C stock for 72 hours at 30°C in 100 ml LB medium supplied with 1% glucose. Cultures were diluted 50, 200, 1000 and 4000-fold in a 1 litre bottle filled with LB medium supplied with 1% glucose. Growth was performed at 30°C for a set time that had been calculated to ensure a logarithmic culture and a stationary phase culture at the aimed time of harvesting.

### Analysis of β-galactosidase Activity in the Chosen Strains

To analyse the β-galactosidase activity, cells were centrifuged at 5000 x g at 4°C for 15 minutes. Pellets were re-dissolved in 1 % of the original volume using a phosphate buffer B (50 mM Na2HPO4.2H2O, 1 mM MgCl2) and then eight 1250 µl aliquots of each cell-free extract transferred to a deep well plate.

The pellets were subsequently lysed using 0.5 gram silicabeads (0.1mm) in 800 µl 0.05M NaPi buffer pH=7.0 and 4 repetitions of 30 second bursts in a cell disruptor. The lysed pellets of the same cell-free extract were then recombined in a single 15 ml Geiner-tube. Cultures were centrifuged for 10 minutes at 5000 x g after the indicated time-period using a 96-well plate centrifuge. 20 µl of supernatant of the cell lysate was dissolved in 180 µl phosphate buffer A (8.9 gr/l Na2HPO4.2H2O, 6.9 gram/l Na2HPO4.H2O, 1mM DTT).

Additionally 10, 100 and 100 fold dilutions of the cell lysate phosphate buffer mix were prepared, to which an ONPG stock solution (20 mM in phopshate buffer) to a starting concentration of 1 mM was added. The absorbance at 420 nm was observed over time using a Pharmacia Biotech Ultrospec 2000 UV/visible spectrophotometer using Swift II Application software and the Miller Units were calculated using the above indicated dilutions.

### GOS Synthesis Protocol

Activity was normalized to 2 mM/min in a total volume of 10 ml by dilution using phsopahe buffer B. 15 ml Greiner tubes were pre-warmed which contained 13.5 ml phosphate buffer B at 30°, 50°, and 60°C. The reaction was started by the addition of 1.5 ml cell-free extract (2 mM/min β-galactosidase activity) to the pre-warmed Greiner tubes. The reactions proceeded with a 30 second time interval. 1 ml samples were then transferred to an Eppendorf tube at 0, 30, 60, 90, 120, 180, 240, 300, and 1440 minute intervals. The GOS formation reaction was then stopped by incubation at 100°C for 5 minutes and the samples immediately stored at -80°C.

Based on the activities of the β-galactosidases found, the actual activity for the GOS formation rate could be predicted. Conversion factors were calculated for each species.

Table 2 below shows the predicted GOS formation rate at 30°C.

**Table 2**

| **Strain** | | | **Bgal activity (Miller Units)** | | | | |
|---|---|---|---|---|---|---|---|
| | **Species** | **Subspecies** | **Average** | **Stdev** | **Used conversion factor** | **Correction factor** | **Predicted GOS formation rate** |
| **no.** | | | **µmol/min/OD-Unit** | | | | **mM/min/100 OD units** |
| 4204 | *Propionibacterium acidipropionici* | | 6.0 | 1.5 | 10.8 | 5.0 | 0.025 |
| 380 | *Propionibacterium sp.* | | 5.6 | 0.4 | 10.8 | 5.0 | 0.023 |
| 2166 | *Propionibacterium freudenreichii* | *freudenreichii* | 4.8 | 0.7 | 10.8 | 5.0 | 0.020 |
| 359 | *Propionibacterium sp.* | | 4.5 | 0.1 | 10.8 | 5.0 | 0.019 |
| 1134 | *Propionibacterium freudenreichii* | *shermanii* | 4.2 | 0.3 | 10.8 | 5.0 | 0.018 |
| 364 | *Propionibacterium jensenii* | | 3.7 | 0.4 | 10.8 | 5.0 | 0.015 |
| 2060 | *Propionibacterium freudenreichii* | *shermanii* | 3.5 | 0.1 | 10.8 | 5.0 | 0.015 |
| 4199 | *Propionibacterium acidipropionici* | | 3.5 | 0.1 | 10.8 | 5.0 | 0.015 |
| 4201 | *Propionibacterium acidipropionici* | | 3.3 | 0.1 | 10.8 | 5.0 | 0.014 |
| 2175 | *Propionibacterium freudenreichii* | *freudenreichii* | 3.3 | 0.2 | 10.8 | 5.0 | 0.014 |
| 2145 | *Propionibacterium freudenreichii* | *freudenreichii* | 3.3 | 0.2 | 10.8 | 5.0 | 0.014 |
| 2168 | *Propionibacterium freudenreichii* | *freudenreichii* | 3.1 | 0.6 | 10.8 | 5.0 | 0.013 |
| 2174 | *Propionibacterium freudenreichii* | *freucfenreichii* | 3.1 | 0.6 | 10.8 | 5.0 | 0.013 |

Table 3 below shows the predicted GOS formation rate at 50°C.

**Table 3**

| **Strain** | | | **Bgal activity (Miller Units)** | | | | |
|---|---|---|---|---|---|---|---|
| | **Species** | **Subspecies** | **Average** | **Stdev** | **Used conversion factor** | **Correction factor** | **Predicted GOS formation rate** |
| **no.** | | | **µmol/min/OD-Unit** | | | | **mM/min/100 OD units** |
| 4204 | *Propionibacterium acidipropionici* | | 6.0 | 1.5 | 10.8 | 5.0 | 0.025 |
| 380 | *Propionibacterium sp.* | | 5.6 | 0.4 | 10.8 | 5.0 | 0.023 |
| 2166 | *Propionibacterium freudenreichii* | *freudenreichii* | 4.8 | 0.7 | 10.8 | 5.0 | 0.020 |
| 359 | *Propionibacterium sp.* | | 4.5 | 0.1 | 10.8 | 5.0 | 0.019 |
| 1134 | *Propionibacterium freudenreichii* | *shermanii* | 4.2 | 0.3 | 10.8 | 5.0 | 0.018 |
| 364 | *Propionibacterium jensenii* | | 3.7 | 0.4 | 10.8 | 5.0 | 0.015 |
| 2060 | *Propionibacterium freudenreichii* | *shermanii* | 3.5 | 0.1 | 10.8 | 5.0 | 0.015 |
| 4199 | *Propionibacterium acidipropionici* | | 3.5 | 0.1 | 10.8 | 5.0 | 0.015 |
| 4201 | *Propionibacterium acidipropionici* | | 3.3 | 0.1 | 10.8 | 5.0 | 0.014 |
| 2175 | *Propionibacterium freudenreichii* | *freudenreichii* | 3.3 | 0.2 | 10.8 | 5.0 | 0.014 |
| 2145 | *Propionibacterium freudenreichii* | *freudenreichii* | 3.3 | 0.2 | 10.8 | 5.0 | 0.014 |
| 2168 | *Propionibacterium freudenreichii* | *freudenreichii* | 3.1 | 0.6 | 10.8 | 5.0 | 0.013 |
| 2174 | *Propionibacterium freudenreichii* | *freudenreichii* | 3.1 | 0.6 | 10.8 | 5.0 | 0.013 |

### GOS Analysis Protocol

High-Performance Anion-Exchange Chromatography with Pulsed Amperometric Detection (HPAEC-PAD) was used to undertake the GOS analysis. HPAEC-PAD analyses were performed on a DX-500 BIO-LCsystem (Dionex) equipped with a PAD. Galactooligosaccharide fractions were separated on CarboPac PA1 column with dimensions 250 mm * 4 mm t a flow rate of 1 mL/min at 22°C. A CarboPac PA1 guard column with dimensions 50 * 4 mm i.d. (Dionex) was used for column protection. The eluents used for the analysis were (A) 500 mM NaOAc + 100mMNaOH, (B) 100mMNaOH and (C) Milli-Q water.

Eluents A and B were mixed to form the following gradient: 100% B from 0 to 5 min followed by 0-26% A in 73 min. After each run, the column was washed with 100% A for 6 min and re-equilibrated for 10 min at 100% B. Peak identification occurred on the basis of comparison of peak distribution of the HPLC chromatogram described in J. Agric. Food Chem. 2009, 57, 8488-8495*.* Lactose was used as a standard for elution time normalization.

### Results

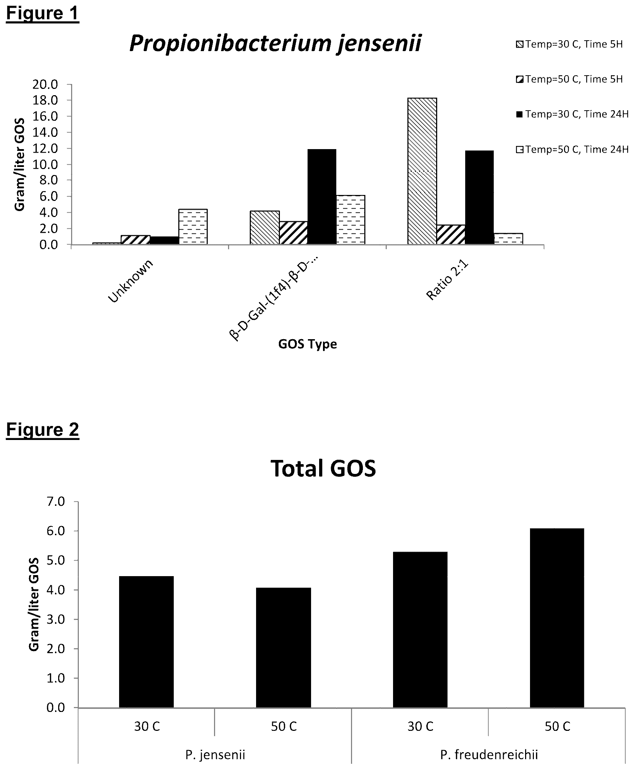

To determine the ratio between highly formed GOS species the most prevalent GOS species for *P. jensenii* were quantified and the ratio between the two species calculated at different temperatures and time points. As shown in Table 4 below and illustrated in Figure 1, it was found that the species ratio showed a strong temperature dependence and a small time dependence.

**Table 4**

| | **Expected GOS linkage type** | **Expected GOS linkage type** | | |
|---|---|---|---|---|
| **Strain** | **Temp** | **Unknown** | **β-D-Gal-(1f4)-β-D-Gal-(1f4)-D-Glc** | **Ratio 2:1** |
| *Propionibacterium jensenii* | Temp=30 C, Time 5H | 0.2 | 4.2 | 18.3 |
| *Propionibacterium jensenii* | Temp=50 C, Time 5H | 1.2 | 2.9 | 2.5 |
| *Propionibacterium jensenii* | Temp=30 C, Time 24H | 1.0 | 12.0 | 11.8 |
| *Propionibacterium jensenii* | Temp=50 C, Time 24H | 4.4 | 6.2 | 1.4 |

Based on standard thermodynamics it was assumed that at 50°C the β-galactosidase reaction occurs at a 4 - 8 times higher rate than at 30°C. For tested samples where the GOS formation rate was at a stage where this was expected to be linear the GOS formation rates were plotted. As shown in Table 5 below and illustrated in Figure 2, no significant increase in activity was detected in either *Propionibacterium* strains.

**Table 5**

| **Strain** | **Temp** | **Total GOS** |
|---|---|---|
| P. jensenii | 30°C | 4.5 |
| | 50°C | 4.1 |

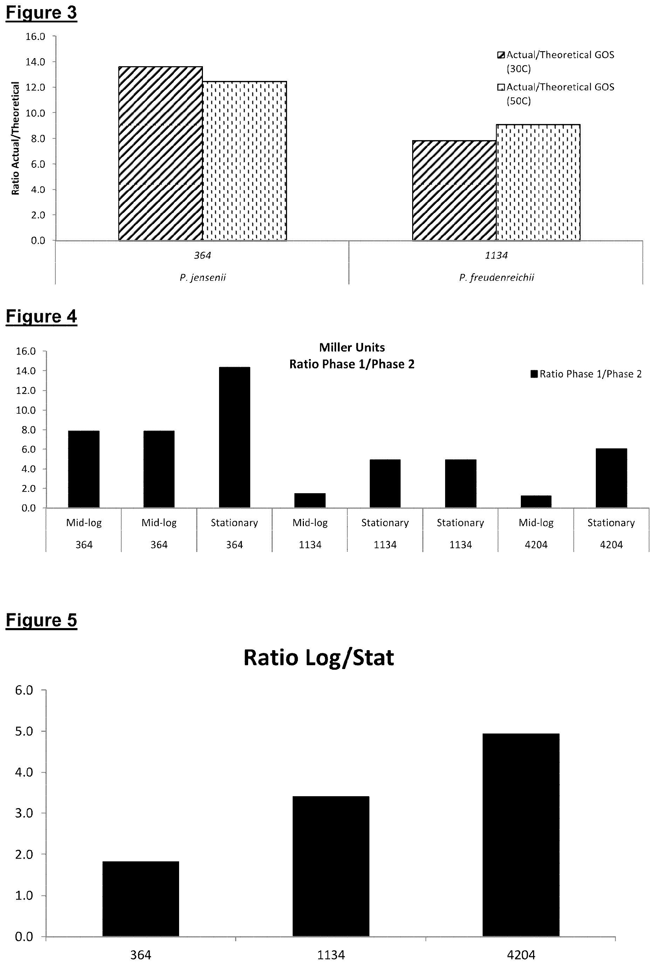

The theoretical GOS formation rate was calculated based on the β-galactosidase activity measured in Miller Units in Phase 2 of the study. Table 6 below shows the ratio of actual GOS formation rate over theoretical GOS formation rate and Figure 3 shows this plotted for both 30°C and 50°C. Surprisingly, and advantageously, GOS formation rates were always found to be higher than the theoretical GOS formation rates.

**Table 6**

| **Strain** | **No** | **Actual/Theoretical GOS (30C)** | **Actual/Theoretical GOS (50C)** | **Ratio** |
|---|---|---|---|---|
| *P. jensenii* | 364 | 13.6 | 12.5 | 0.9 |
| *P. freudenreichii* | 1134 | 7.9 | 9.1 | 1.2 |
| **Average** | | **10.8** | **10.8** | **1.0** |

The β-galactosidase activity analysed in the initial phase of experiments in general appeared to be higher than those activities determined in the later phase. To find out whether there is a consistent error in the methodology the ratios of the activities in phase 1 and 2 were calculated (and shown in Table 7 below) and plotted on a graph shown in Figure 4. Figure 4 shows that for most samples a 5-fold difference is detected. Some samples clearly show much higher differences, and this is expected that these differences are mainly due to the differences in the growth phase of the cells.

**Table7**

| **Strain no** | **Growth Phase** | **Ratio Phase 1/Phase 2** |
|---|---|---|
| 364 | Mid-log | 7.9 |
| 364 | Mid-log | 7.9 |
| 364 | Stationary | 14.4 |
| 1134 | Mid-log | 1.5 |
| 1134 | Stationary | 5.0 |
| 1134 | Stationary | 5.0 |
| 4204 | Mid-log | 1.2 |
| 4204 | Stationary | 6.1 |

To assess whether the expression of β-galactosidase was dependent on the growth phase of the organism, the activity (measured in Miller Units) was plotted for all strains. Table 8 and Figure 5 show the data and plot respectively. It was found that for most strains a Log:Stat ratio ≥ 1 was found indicating that the activity of β-galactosidase is higher in the Log phase than in the stationary phase. These differences are limited and it may be that the higher biomass yield in stationary phase off-sets the lower β-galactosidase activities.

**Table 8**

| | | **Ratio Log/Stat** |
|---|---|---|
| *Propionibacterium jensenii* | 364 | 1.8 |
| *Propionibacterium freudenreichii* | 1134 | 3.4 |
| *Propionibacterium acidipropionici* | 4204 | 4.9 |

### Conclusions

All *Propionibacterium* strains produced GOS and the cell-free extracts showed approximately similar GOS formation rates at 30°C and 50°C. All samples show a different GOS profile than the GOS produced by *Apergillus Oryzea* enzyme. Specifically strain 364 (P. *jensenii*) showed significant GOS production yields. In general, the later GOS synthesis phase showed a 5-fold lower β-galactosidase activities as compared to the initial screening phase.

These experiments showed that it was possible for *Propionibacterium* strains to produce GOS, which could potentially be used as a selective growth medium for a chosen *Propionibacterium* probiotic bacterial strain so to promote growth in the lower gut so as help modulate appetite.

The forgoing embodiments are not intended to limit the scope of the protection afforded by the claims, but rather to describe examples of how the invention may be put into practice.

## Claims

1. A prebiotic composition comprising a galacto oligosaccharide (GOS) produced from one or more *Propionibacterium* bacterial strains, wherein the GOS acts as a selective growth medium for *Propionibacterium* bacterial strains, and wherein the GOS is substantially the same as the form produced by reverse β-galactosidase reaction for *Propionibacterium* bacterial strains.

2. A composition as claimed in claim 1, wherein the GOS is produced and/or is selective for one of more of the following bacterial strains: *Propionibacterium jensenii; Propionibacterium freudenreichii; Propionibacterium acidipropionici,* or sub-species or mutant strains thereof.

3. A composition as claimed in any preceding claim, wherein the (GOS) is produced from a selected *Propionibacterium* bacterial genus or strain and the GOS acts as a selective growth medium for said selected *Propionibacterium* bacterial genus or strain.

4. A composition as claimed in any preceding claim, wherein the composition is encapsulated.

5. A composition as claimed in any preceding claim, wherein the composition further comprises an excipient or carrier compound to enable it to pass through at least part of the gastrointestinal environment of the body and retain its functional properties.

6. A composition as claimed in any preceding claim, wherein the composition is in the form of a liquid or powder and/or can be mixed with a solid or liquid food stuff.

7. A composition as claimed in any one of claims 1 to 5 for use as a medicament.

8. A composition as claimed in claim 7, wherein the medicament is for the treatment and/or management of obesity.

9. A composition as claimed in any one of claims 1 to 5 for use as a dietary supplement.

10. A composition as claimed in any one of claims 1 to 5 for use in the regulation and/or modulation of appetite in an individual.

11. A composition as claimed in any one of claims 1 to 5 for use in weight management.

12. A composition as claimed in any one of claims 1 to 5 for use in increasing propionate levels in the lower gut of an individual wherein the composition promotes the growth of propionate secreting bacteria.

13. A method of producing galactooligosaccharide (GOS) comprising the steps of growing one or more *Propionibacterium* strains in a growth medium comprising up to 50 % lactose at a temperature of up to 55 °C for up to 24 hours under anaerobic conditions and harvesting GOS from the *Propionibacterium* cells.

14. The method as claimed in claim 13, wherein the one or more *Propionibacterium* strains are grown in a growth medium comprising up to 40 % lactose at a temperature of up to 50 °C for up to 24 hours.

15. The method as claimed in any one of claims 13 to 14, wherein the GOS is harvested from the *Propionibacterium* cells by lysis.

16. The method as claimed in any one of claims 13 to 15, wherein the *Propionibacterium* strains are selected from one or more of the following: *Propionibacterium jensenii; Propionibacterium freudenreichii; Propionibacterium acidipropionici,* or sub-species or mutant strains thereof.

17. The method as claimed in any one of claims 13 to 16, wherein the method is used to produce GOS for use in a composition as claimed in any one of claims 1 to 11.

## Patentansprüche

1. Präbiotische Zusammensetzung, umfassend ein Galacto-Oligosaccharid (GOS), hergestellt aus einem oder mehreren *Propionibacterium*-Bakterienstämmen, wobei das GOS als selektives Wachstumsmedium für *Propionibacterium*-Bakterienstämme wirkt und wobei das GOS im Wesentlichen das gleiche ist wie die von der reversen β-Galactosidase-Reaktion für *Propionibacterium*-Bakterienstämme produzierte Form.

2. Zusammensetzung nach Anspruch 1, wobei das GOS produziert wird und/oder selektiv ist für einen oder mehrere der folgenden Bakterienstämme: *Propionibacterium jensenii; Propionibacterium freudenreichii; Propionibacterium acidipropionici* oder Unterarten oder Mutantenstämme davon.

3. Zusammensetzung nach einem der vorhergehenden Ansprüche, wobei das (GOS) produziert wird aus einer/einem ausgewählten *Propionibacterium*-Bakteriengattung oder -stamm und das GOS als ein selektives Wachstumsmedium für die/den ausgewählten *Propionibacterium*-Bakteriengattung oder -stamm wirkt.

4. Zusammensetzung nach einem der vorhergehenden Ansprüche, wobei die Zusammensetzung eingekapselt ist.

5. Zusammensetzung nach einem der vorhergehenden Ansprüche, wobei die Zusammensetzung ferner einen Exzipienten oder eine Trägerverbindung umfasst, um ihr zu ermöglichen, durch wenigstens einen Teil der gastrointestinalen Umgebung des Körpers hindurchzulaufen und ihre funktionellen Eigenschaften beizubehalten.

6. Zusammensetzung nach einem der vorhergehenden Ansprüche, wobei die Zusammensetzung in Form einer Flüssigkeit oder eines Pulvers vorliegt und/oder mit einem festen oder flüssigen Nahrungsmittel gemischt werden kann.

7. Zusammensetzung nach einem der Ansprüche 1 bis 5 zur Verwendung als Medikament.

8. Zusammensetzung nach Anspruch 7, wobei das Medikament zur Behandlung und/oder Reduktion von Fettleibigkeit dient.

9. Zusammensetzung nach einem der Ansprüche 1 bis 5 zur Verwendung als Nahrungsergänzungsmittel.

10. Zusammensetzung nach einem der Ansprüche 1 bis 5 zur Verwendung bei der Regulierung und/oder Modulation des Appetits in einem Individuum.

11. Zusammensetzung nach einem der Ansprüche 1 bis 5 zur Verwendung in der Gewichtsreduktion.

12. Zusammensetzung nach einem der Ansprüche 1 bis 5 zur Verwendung bei der Erhöhung der Propionatspiegel im unteren Darm eines Individuums, wobei die Zusammensetzung das Wachstum von Propionat-sezernierenden Bakterien fördert.

13. Verfahren zur Herstellung von Galactooligosaccharid (GOS), umfassend die Schritte des Heranzüchtens eines oder mehrerer *Propionibacterium*-Stämme in einem Wachstumsmedium, umfassend bis zu 50% Lactose, bei einer Temperatur von bis zu 55°C während bis zu 24 Stunden unter anaeroben Bedingungen und des Erntens von GOS aus den *Propionibacterium*-Zellen.

14. Verfahren nach Anspruch 13, wobei der eine oder die mehreren *Propionibacterium*-Stämme in einem Wachstumsmedium, das bis bis zu 40% Lactose umfasst, bei einer Temperatur von bis zu 50°C während bis zu 24 Stunden herangezüchtet werden.

15. Verfahren nach einem der Ansprüche 13 bis 14, wobei das GOS aus den *Propionibacterium*-Zellen durch Lyse geerntet wird.

16. Verfahren nach einem der Ansprüche 13 bis 15, wobei die *Propionibacterium*-Stämme ausgewählt sind aus einem oder mehreren der folgenden: *Propionibacterium jensenii; Propionibacterium freudenreichii; Propionibacterium acidipropionici* oder Unterarten oder Mutantenstämme davon.

17. Verfahren nach einem der Ansprüche 13 bis 16, wobei das Verfahren zur Herstellung von GOS zur Verwendung in einer Zusammensetzung nach einem der Ansprüche 1 bis 11 verwendet wird.

## Revendications

1. Composition prébiotique comprenant un galactooligosaccharide (GOS) produit à partir d'une ou plusieurs souches bactériennes de *Propionibacterium,* dans laquelle le GOS agit en tant que milieu de culture sélectif pour les souches bactériennes de *Propionibacterium,* et dans laquelle le GOS est sensiblement identique à la forme produite par réaction de β-galactosidase inverse pour les souches bactériennes de *Propionibacterium.*

2. Composition selon la revendication 1, dans laquelle le GOS est produit et/ou est sélectif pour une ou plusieurs des souches bactériennes suivantes : *Propionibacterium jensenii ; Propionibacterium freudenreichii ; Propionibacterium acidipropionici,* ou des sous-espèces ou des souches mutantes de celles-ci.

3. Composition selon l'une quelconque des revendications précédentes, dans laquelle le (GOS) est produit à partir d'un genre ou d'une souche bactérien (ne) de *Propionibacterium* et le GOS agit en tant que milieu de culture sélectif pour ledit genre ou ladite souche bactérien(ne) sélectionné(e) de Propionibacterium.

4. Composition selon l'une quelconque des revendications précédentes, la composition étant encapsulée.

5. Composition selon l'une quelconque des revendications précédentes, la composition comprenant en outre un composé d'excipient ou véhicule pour permettre à celle-ci de traverser au moins une partie de l'environnement gastro-intestinal du corps et conserver ses propriétés fonctionnelles.

6. Composition selon l'une quelconque des revendications précédentes, la composition étant sous la forme d'un liquide ou d'une poudre et/ou pouvant être mélangée avec un aliment solide ou liquide.

7. Composition selon l'une quelconque des revendications 1 à 5 pour utilisation en tant que médicament.

8. Composition selon la revendication 7, le médicament étant pour le traitement et/ou le contrôle de l'obésité.

9. Composition selon l'une quelconque des revendications 1 à 5, pour utilisation en tant que complément alimentaire.

10. Composition selon l'une quelconque des revendications 1 à 5, pour utilisation dans la régulation et/ou la modulation de l'appétit chez un individu.

11. Composition selon l'une quelconque des revendications 1 à 5 pour utilisation dans le contrôle du poids.

12. Composition selon l'une quelconque des revendications 1 à 5 pour utilisation dans l'augmentation des taux de propionate dans l'intestin inférieur d'un individu, la composition stimulant la croissance de bactéries sécrétant du propionate.

13. Procédé de production de galactooligosaccharide (GOS) comprenant les étapes de culture d'une ou plusieurs souches de *Propionibacterium* dans un milieu de culture comprenant jusqu'à 50 % de lactose à une température allant jusqu'à 55 °C pendant jusqu'à 24 heures dans des conditions anaérobies et collecte de GOS à partir des cellules de *Propionibacterium.*

14. Procédé selon la revendication 13, dans lequel les une ou plusieurs souches de *Propionibacterium* sont cultivées dans un milieu de culture comprenant jusqu'à 40 % de lactose à une température allant jusqu'à 50 °C pendant jusqu'à 24 heures.

15. Procédé selon l'une quelconque des revendications 13 à 14, dans lequel le GOS est collecté à partir des cellules de *Propionibacterium* par lyse.

16. Procédé selon l'une quelconque des revendications 13 à 15, dans lequel les souches de *Propionibacterium* sont choisies parmi l'une ou plusieurs des souches suivantes : *Propionibacterium jensenii ; Propionibacterium freudenreichii ; Propionibacterium acidipropionici,* ou des sous-espèces ou des souches mutantes de celles-ci.

17. Procédé selon l'une quelconque des revendications 13 à 16, le procédé étant utilisé pour produire un GOS pour utilisation dans une composition selon l'une quelconque des revendications 1 à 11.
